# EUROPEAN PATENT SPECIFICATION

(11) **EP 1 737 490 B1**
(45) Date of publication and mention of the grant of the patent: **09.06.2010**
(21) Application number: 05735998.6
(22) Date of filing: 06.04.2005
(51) Int. Cl.: A61K 39/395, A61P 37/00, A61P 9/10

(54) **METHODS OF TREATING ATHEROSCLEROSIS**
VERFAHREN ZUR BEHANDLUNG VON ATHEROSKLEROSE
PROCEDES DE TRAITEMENT D'ATHEROSCLÉROSE

(30) Priority: 06.04.2004 US 559944 P
(43) Date of publication of application: 03.01.2007
(73) Proprietor: NovImmune S.A., 1211 Genève 4 (CH)
(72) Inventor: MACH, Bernard, CH-1292 Chambésy (CH); DEAN, Yann, F-74580 Viry (FR); KOSCO-VILBOIS, Marie, F-74270 Minzier (FR)
(74) Representative: Peter, Beate
(86) International application number: PCT/US2005/011767
(87) International publication number: WO 2005/099755

(56) References cited:
- GB-A- 2 380 127
- US-A- 6 025 477
- US-A1- 2003 216 551
- G. TRAN ET AL.: "Reversal of experimental allergic encephalomyelitis with non-mitogenic, non-depleting anti-CD3 mAb therapy with a preferential effect on Th1 cells that is augmented by IL-4." INTERNATIONAL IMMUNOLOGY, vol. 13, no. 9, 2001, pages 1109-1120, XP002365311
- Z. BILINSKA ET AL.: "Active lymphocytic myocarditis treated with murine OKT3 monoclonal antibody." TEXAS HEART INSTITUTE JOURNAL, vol. 29, no. 2, 2002, pages 113-117, XP002365312
- M. VON HERRATH ET AL.: "Nonmitogenic CD3 antibody reverses virally induced (rat insulin promoter-lymphocytic choriomenigitis virus) autoimmune diabetes without impeding viral clearance." THE JOURNAL OF IMMUNOLOGY, vol. 168, no. 2, 15 January 2002 (2002-01-15), pages 933-941, XP002365313
- K. HEROLD ET AL.: "Activation of human T cells by FcR nonbinding anti-CD3 mAb hOKT3gamma1(Ala-Ala)." THE JOURNAL OF CLINICAL INVESTIGATION, vol. 111, no. 3, February 2003 (2003-02), pages 409-418, XP002365314

## Description

### FIELD OF THE INVENTION

This invention relates generally to the use of CD3 modulators, such as anti-CD3 antibodies, to induce immunological tolerance and modulation of the immune response for the treatment of autoimmune diseases and/or inflammatory disorders.

### BACKGROUND OF THE INVENTION

The immune system is highly complex and tightly regulated, with many alternative pathways capable of compensating deficiencies in other parts of the system. There are however occasions when the immune response becomes a cause of disease or other undesirable conditions if activated. Such diseases or undesirable conditions are, for example, autoimmune diseases, graft rejection after transplantation, allergy to innocuous antigens, psoriasis, chronic inflammatory diseases such as atherosclerosis, and inflammation in general. In these cases and others involving inappropriate or undesired immune response, there is a clinical need for immunosuppression.

Accordingly, there exists a need for compositions that can be used in the treatment of immune-related diseases and/or disorders.

### SUMMARY OF THE INVENTION

The invention is based on the discovery that a modulation of CD3 expression or activity, leads to a decrease in atherosclerotic plaque development in a mouse model for clinical atherosclerosis. Accordingly, the invention features methods of preventing or inhibiting inflammation in a bodily tissue, wherein said tissue is vascular tissue. An inflamed tissue is characterized generally by redness, pain and swelling of the tissue. The tissue includes cardiac tissue, *e.g*., a vein, an artery, or a capillary.

Inflammation is inhibited by exposing a cell or tissue to a CD3 modulator in an amount that leads to a reduction in the production of a proinflammatory cytokine, an increase in an anti-inflammatory cytokine or in an amount that leads to immunological tolerance.

The invention provides methods of inhibiting the formation of atherosclerotic plaque by exposing a CD3 expressing cell to a CD3 modulator. Atherosclerotic plaque is inhibited such that the amount of plaque associated with the arterial wall is reduced after exposure to the CD3 modulator compared to before exposure to the CD3 inhibitor.

The cell is any cell that is capable of expressing CD3, *e.g*., a lymphocyte such as a T-cell. The T-cell is a circulating T-cell, *i.e.,* in the blood or lymph. Alternatively, the T-cell is within a tissue, *e.g*. a lymph node, lymph ducts, lymph vessels and spleen. The tissue is an inflamed tissue (or a tissue that is at risk of becoming inflamed). By exposing is meant that the cell or tissue is contacted with the CD3 modulator. The cell of tissue is contacted directly or indirectly (*i.e*., systemically). The cell is contacted *in vivo, in vitro,* or *ex vivo.*

The invention also features methods of preventing or alleviating a symptom of an inflammatory disorder, wherein said inflammatory disorder is atherosclerosis, by administering to the subject a CD3 modulator.

A CD3 modulator is a compound that decreases the expression or activity of CD3. CD3 activities include T-cell activation. Methods of measuring T-cell activation are well known in the art. CD3 modulators include, for example, anti-CD3 antibodies. CD3 modulators are administered alone or in combination with another anti-inflammatory agent or immunosuppressive drugs used to treat an inflammatory disorder. For example, CD3 modulator is administered with corticosteroid, a statin, interferon beta, a nonsteroidal anti-inflammatory drugs (NSAIDs), methotrexate, Cyclosporin A or a disease-modifying anti-rheumatic drugs (DMARDs).

This invention specifically relates to use of an anti-CD3 antibody for the manufacture of a medicament for treating or alleviating a symptom of an inflammatory disorder in a subject wherein said inflammatory disorder is atherosclerosis, and for reducing the formation of atherosclerotic plaque on an artery.

This invention also specifically relates to an anti-CD3 antibody for use in treating or alleviating a symptom of an inflammatory disorder, wherein said inflammatory disorder is atherosclerosis.

The subject is a mammal such as human, mouse, rat, dog, cat, cow, horse, and pig. The subject is suffering from or at risk of developing an inflammatory disorder. Inflammatory disorders include cardiovascular inflammation, ocular inflammation, gastrointestinal inflammation, hepatic inflammation, pulmonary inflammation, autoimmune disorders or muscular inflammation. A subject suffering from or at risk of developing inflammatory disorder is identified by methods known in the art, *e.g*., gross examination of tissue or detection of inflammation associated in tissue or blood. Symptoms of inflammation include pain, redness and swelling of the affected tissue.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In the case of conflict, the present specification, including definitions, will control. In addition, the materials, methods, and examples are illustrative only and are not intended to be limiting.

Other features and advantages of the invention will be apparent from the following detailed description and claims.

### BRIEF DESCRIPTION OF THE FIGURES

Figure 1 is a graph depicting the clinical and histological effects of anti-CD3 therapy in a rat uveitis model.
Figure 2 is a graph depicting the inhibitory effect of anti-CD3 therapy on atherosclerotic plaque development in the aortic root of mice.
Figure 3 is a graph depicting the inhibitory effect of anti-CD3 therapy on the progression of established atherosclerotic lesions in mice.

### DETAILED DESCRIPTION OF THE INVENTION

The invention is based in part on the discovery that a modulation of CD3 expression or activity results in a reduced immune response and an anti-inflammatory effect. More specifically, administration of an anti-CD3 antibody resulted in a reduction of signs and symptoms associated with uveitis and a reduction of atherosclerotic plaque development.

CD3 is a complex of at least five membrane-bound polypeptides in mature T-lymphocytes that are non-covalently associated with one another and with the T-cell receptor. The CD3 complex includes the gamma, delta, epsilon, zeta, and eta chains (also referred to as subunits). When antigen binds to the T-cell receptor, the CD3 complex transduces the activating signals to the cytoplasm of the T-cell.

### CD3 Modulators

A CD3 modulator refers to an agent that modulates the expression or activity of CD3. By modulates is meant, the agent promotes, increases, decrease or neutralizes the expression or activity of CD3. A CD3 activity includes, for example, transducing a T-cell activation signal. T-cell activation is defined by an increase in calcium mediated intracellular cGMP or an increase in cell surface receptors for IL-2. For example, a increase of T-cell activation is characterized by a decrease of calcium- mediated intracellular cGMP and or IL-2 receptors in the presence of the compound compared to a the absence of the compound. Intracellular cGMP is measured, for example, by a competitive immunoassay or scintillation proximity assay using commercially available test kits. Cell surface IL-2 receptors are measured for example, by determining binding to an IL-2 receptor antibody such as the PC61 antibody.

A CD3 modulator includes, for example anti- CD3 antibodies or fragments thereof. Optionally, the anti-CD3 antibody is a single chain anti-CD3 antibody, a bispecific anti-CD3 antibody or a heteroconjugate anti-CD3 antibody. The antibody is an activating anti-CD3 antibody, thus, it induces CD3 activity. Alternatively, the antibody is a neutralizing anti-CD3 antibody, thus, it reduces CD3 activity.
Anti-CD3 antibodies are well known in the art. Exemplary anti-CD3 antibodies include, but are not limited to, OKT3, G4.18, 145-2C11, Leu4, HIT3a, BC3, SK7, SP34, RIV-9, and UCHT1. Preferably, the anti-CD3 antibody binds the same epitopes as OKT3, G4.18, 145-2C11, Leu4, HIT3a, BC3, SK7, SP34, RIV-9, and UCHT1. Preferably, the antibody has been humanized or equinized as to reduce the host's immune response to the antibody.

Those skilled in the art will recognize that it is possible to determine, without undue experimentation, if an antibody has the same epitopes as anti-CD3 antibody OKT3, G4.18, 145-2C11, Leu4, HIT3a, BC3, SK7, SP34, RIV-9, and UCHT1 by ascertaining whether the former prevents the latter from binding to a CD3 antigen polypeptide or other T cell surface antigen polypeptide. The CD3 antigen polypeptide is for example the CD3 epsilon polypeptide complexed with another CD3 subunit polypeptide (*e.g*., gamma, delta, epsilon, zeta or eta chains.) If the antibody being tested competes with an antibody of the invention, as shown by a decrease in binding by the antibody of the invention, then the two antibodies bind to the same, or a closely related, epitope. Another way to determine whether an antibody has the specificity of an antibody of the invention is to pre-incubate the antibody of the invention with the CD3 antigen polypeptide or T cell surface antigen polypeptide with which it is normally reactive, and then add the antibody being tested to determine if the antibody being tested is inhibited in its ability to bind the CD3 or T cell surface antigen polypeptide. If the antibody being tested is inhibited then, in all likelihood, it has the same, or functionally equivalent, epitopic specificity as the antibody of the invention.

As used herein, the term "antibody" refers to immunoglobulin molecules and immunologically active portions of immunoglobulin (Ig) molecules, *i.e*., molecules that contain an antigen binding site that specifically binds (immunoreacts with) an antigen. Such antibodies include, polyclonal, monoclonal, chimeric, single chain, F_{ab}, F_{ab'} and F_{(ab')2} fragments, and an F_{ab} expression library. Preferably, the antibody is a fully human antibody. By "specifically bind" or "immunoreacts with" is meant that the antibody reacts with one or more antigenic determinants of the desired antigen and does not react *(i.e.,* bind) with other polypeptides or binds at much lower affinity (K_{d} > 10⁻⁶) with other polypeptides.

As used herein, the term "epitope" includes any protein determinant capable of specific binding to an immunoglobulin, an scFv, or a T-cell receptor. The term "epitope" includes any protein determinant capable of specific binding to an immunoglobulin or T-cell receptor. Epitopic determinants usually consist of chemically active surface groupings of molecules such as amino acids or sugar side chains and usually have specific three dimensional structural characteristics, as well as specific charge characteristics. An antibody is said to specifically bind an antigen when the dissociation constant is ≤ 1 µM; preferably ≤ 100 nM and most preferably ≤ 10 nM.

As used herein, the terms "immunological binding," and "immunological binding properties" refer to the non-covalent interactions of the type that occur between an immunoglobulin molecule and an antigen for which the immunoglobulin is specific. The strength, or affinity of immunological binding interactions can be expressed in terms of the dissociation constant (K_{d}) of the interaction, wherein a smaller K_{d} represents a greater affinity. Immunological binding properties of selected polypeptides are quantified using methods well known in the art. One such method entails measuring the rates of antigen-binding site/antigen complex formation and dissociation, wherein those rates depend on the concentrations of the complex partners, the affinity of the interaction, and geometric parameters that equally influence the rate in both directions. Thus, both the "on rate constant" (Kₒₙ) and the "off rate constant" (K_{off}) can be determined by calculation of the concentrations and the actual rates of association and dissociation. (*See* Nature 361:186-87 (1993)). The ratio of K_{off}/Kₒₙ enables the cancellation of all parameters not related to affinity, and is equal to the dissociation constant K_{d}. (*See, generally,* Davies et al. (1990) Annual Rev Biochem 59:439-473). An antibody of the present invention is said to specifically bind to a CD3 epitope when the equilibrium binding constant (K_{d}) is ≤1 µM, preferably ≤ 100 nM, more preferably ≤ 10 nM, and most preferably ≤ 100 pM to about 1 pM, as measured by assays such as radioligand binding assays or similar assays known to those skilled in the art.

It is desirable to modify the antibody of the invention with respect to effector function, so as to enhance the effectiveness of the antibody in treating immune-related diseases. For example, cysteine residue(s) can be introduced into the Fc region, thereby allowing interchain disulfide bond formation in this region. The homodimeric antibody thus generated can have improved internalization capability and/or increased complement-mediated cell killing and antibody-dependent cellular cytotoxicity (ADCC). (*See* Caron et al J. Exp Med., 176: 1191-1195 (1992) and Shopes, J. Immunol., 148: 2918-2922 (1992)). Alternatively, an antibody can be engineered that has dual Fc regions and can thereby have enhanced complement lysis and ADCC capabilities. (*See* Stevenson et al., Anti-Cancer Drug Design, 3: 219-230 (1989)).

Antibodies are purified by well-known techniques, such as affinity chromatography using protein A or protein G, which provide primarily the IgG fraction of immune serum. Subsequently, or alternatively, the specific antigen which is the target of the immunoglobulin sought, or an epitope thereof, may be immobilized on a column to purify the immune specific antibody by immunoaffinity chromatography. Purification of immunoglobulins is discussed, for example, by D. Wilkinson (The Scientist, published by The Scientist, Inc., Philadelphia PA, Vol. 14, No. 8 (April 17, 2000), pp. 25-28).

### Therapeutic Methods

Inflammation is inhibited by exposing, *e.g*., contacting a tissue or a CD3 expressing cell with a CD3 modulator, *e.g*., an anti-CD3 antibody. A CD3 expressing cell is for example, a lymphocyte such as a T-cell. T cells include T cytotoxic cells, T helper cells (*e.g*., Th1 and Th2) and natural killer T-cells. Tissues to be treated include a gastrointestinal tissue, *e.g*., an intestinal tissue, a cardiac tissue, *e.g*., a vein, artery or capillary, a pulmonary tissue, a dermal tissue, an ocular tissue, *e.g*., iris, the ciliary body, or the choroid, or a hepatic tissue.

Inhibition of inflammation can be characterized by a reduction of redness, pain and swelling of the treated tissue compared to a tissue that has not been in contact with a CD3 modulator. Alternatively, with respect to uveitis, inhibition of inflammation is characterized by increased visual acuity, and decreased cells and turbidity (fibrin, denatured proteins) in vitreous (*i.e*., posterior part of the eye) and anterior chamber of the eye. Tissues or cells are directly contacted with a CD3 modulator. Alternatively, the CD modulator is administered systemically. CD3 modulators are administered in an amount sufficient to decrease (*e.g*., inhibit) pro-inflammatory cytokine production. A pro-inflammatory cytokine is a cytokine that induces an inflammatory response. A pro-inflammatory cytokine includes, for example, interleukin (IL)-1, tumor necrosis factor (TNF), IL-17, IL-8, and macrophage inflammatory protein-1 alpha (MIP-1 alpha). Alternatively, the CD3 modulators are administered in an amount sufficient to increase (*e.g*., promote) anti-inflammatory cytokine production. An anti-inflammatory cytokine is a cytokine that reduces an inflammatory response. More specifically, an anti-inflammatory cytokine controls the proinflammatory cytokine response to regulate the immune response. Anti-inflammatory cytokines include, for example, the interleukin (IL)-1 receptor antagonist, TGFβ, IL-4, IL-6, IL-10, IL-11, and IL-13. Cytokines are detected for example in the serum, plasma or the tissue. Cytokine production is measured by methods known in the art. For example, cytokine production is determined using an immunoassay specific for a pro-inflammatory cytokine or an anti-inflammatory cytokine.

An inflammatory response is evaluated morphologically by observing tissue damage, localized redness, swelling of the affected area, cells and turbidity in the vitreous and anterior chamber of the eye. Alternatively, an inflammatory response is evaluated by measuring c-reactive protein, or IL-1 in the tissue, serum or plasma. A return to baseline white blood count also indicates a decrease in inflammation.

Alternatively, the inflammation is reduced by exposing a tissue or a CD3 expressing cell to a CD3 modulator in an amount to redistribute and/or eliminate the CD3-T cell receptor complex or CD3 complex on the surface of a cell, *e.g*., a lymphocyte. For example, the CD3 expressing cell is exposed with a CD3 modulator in an amount to modulate, *i.e*., reduce the CD3 expressing cell-cell contact. Decrease in the level of cell surface expression or activity of the TcR on the cell is meant that the amount or function of the TcR is reduced. Modulation of the level of cell surface expression or activity of CD3 is meant that the amount of CD3 on the cell surface or function of CD3 is altered, *e.g*., reduced. The amount of CD3 or the TcR expressed at the plasma membrane of the cell is reduced, for example, by internalization of CD3 or the TcR upon contact of the cell with the CD3 modulator. Alternatively, upon contact of a cell with the CD3 modulator CD3 is masked. Redistribution and/or elimination of the CD3-T cell receptor complex on the surface of a cell results in immunological suppression or immune tolerance. Immunological suppression is the failure to mount a general immune response to all antigens, resulting in the alleviation of one or more symptoms of an autoimmune disorder or a decrease in the inflammatory response. Immunological suppression is reversible once treatment with the CD3 modulator is withdrawn. Thus, immunological suppression is particularly useful in treating or alleviating a symptom of a acute inflammatory disorder. In contrast, immune tolerance is the failure to mount an immune response to a specific antigen, resulting in the alleviation of one or more symptoms of an autoimmune disorder or a decrease in the inflammatory response that is maintained long term once treatment is withdrawn. Immunological tolerance is maintained for at least 1 week, 1 month, 3 months, 6 months, 1 year, 2 years, or 5 or more years after treatment is withdrawn. Thus, immunological tolerance is particularly useful in treating or alleviating a symptom of a chronic inflammatory disorder. Long term immunological tolerance can be achieved either through soluble mechanisms such as cytokines or via direct cell-cell contact involving but not limited to Granzyme A, Granzyme B and/or Perforin.

The formation of atherosclerotic plaque on an artery, *i.e*. arterial wall, is reduced or prevented by administering to a subject a CD3 modulator. Plaque is a combination of cholesterol, other fatty materials, calcium, and blood components that accumulate within the artery wall lining. A reduction of atherosclerotic plaque is defined by a decrease in luminal narrowing, *i.e*., a widening of the lumen. Luminal width is measured by methods known in the art, such as contrast angiography and Doppler velocity waveform analysis.

The methods are useful to alleviate the symptoms of a variety of inflammatory disorders or autoimmune disorders. The inflammatory disorder is acute or chronic. Inflammatory disorders include cardiovascular inflammation (*e.g*., atherosclerosis, stroke), gastrointestinal inflammation, hepatic inflammatory disorders, pulmonary inflammation (*e.g*. asthma, ventilator induced lung injury), kidney inflammation, ocular inflammation (*e.g*., uveitis), pancreatic inflammation, genitourinary inflammation, neuroinflammatory disorders (*e.g*., multiple sclerosis, Alzheimer's disease), allergy (*e.g.,* allergic rhinitis/sinusitis, skin allergies and disorders (*e.g.,* urticaria/hives, angioedema, atopic dermatitis, contact dermatitis, psoriasis), food allergies, drug allergies, insect allergies, mastocytosis), skeletal inflammation (*e.g*., arthritis, osteoarthritis, rheumatoid arthritis, spondyloatthropathies), infection (*e.g*., bacterial or viral infections ; oral inflammatory disorders (*i.e*., perodontis, gingivitis or somatitis); and transplantation (*e.g*., allograft or xenograft rejection or maternal-fetal tolerance).

Autoimmune diseases include, for example, Acquired Immunodeficiency Syndrome (AIDS, which is a viral disease with an autoimmune component), alopecia areata, ankylosing spondylitis, antiphospholipid syndrome, autoimmune Addison's disease, autoimmune hemolytic anemia, autoimmune hepatitis, autoimmune inner ear disease (AIED), autoimmune lymphoproliferative syndrome (ALPS), autoimmune thrombocytopenic purpura (ATP), Behcet's disease, cardiomyopathy, celiac sprue-dermatitis hepetiformis; chronic fatigue immune dysfunction syndrome (CFIDS), chronic inflammatory demyelinating polyneuropathy (CIPD), cicatricial pemphigold, cold agglutinin disease, crest syndrome, Crohn's disease, Degos' disease, dermatomyositis-juvenile, discoid lupus, essential mixed cryoglobulinemia, fibromyalgiafibromyositis, Graves' disease, Guillain-Barré syndrome, Hashimoto's thyroiditis, idiopathic pulmonary fibrosis, idiopathic thrombocytopenia purpura (ITP), IgA nephropathy, insulin-dependent diabetes mellitus, juvenile chronic arthritis (Still's disease), juvenile rheumatoid arthritis, Ménière's disease, mixed connective tissue disease, multiple sclerosis, myasthenia gravis, pernacious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomena, Reiter's syndrome, rheumatic fever, rheumatoid arthritis, sarcoidosis, scleroderma (progressive systemic sclerosis (PSS), also known as systemic sclerosis (SS)), Sjögren's syndrome, stiff-man syndrome, systemic lupus erythematosus, Takayasu arteritis, temporal arteritis/giant cell arteritis, ulcerative colitis, uveitis, vitiligo and Wegener's granulomatosis.

The methods described herein lead to a reduction in the severity or the alleviation of one or more symptoms of an inflammatory disorder or an autoimmune disorder such as those described herein. Efficacy of treatment is determined in association with any known method for diagnosing or treating the particular inflammatory disorder. Alleviation of one or more symptoms of the inflammatory disorder or autoimmune disease indicates that the compound confers a clinical benefit. Inflammatory disorders and autoimmune disorders are diagnosed and/or monitored, typically by a physician or veterinarian using standard methodologies.

### Uveitis

Uveitis defined as inflammation of the uvea. The uvea consists of three structures: the iris, the ciliary body, and the choroid. The iris is the colored structure surrounding the pupil, visible in the front of the eye. The ciliary body is a structure containing muscle and is located behind the iris which focuses the lens. The choroid is a layer containing blood vessels that line the back of the eye and is located between the retina and the osclera. Symptoms of uveitis include reduced visual acuity, cells and turbidity (fibrin, denatured proteins) in the vitreous and anterior chamber of the eye and vasculitis of retinal vessels. The iris may adhere to the lens capsule (posterior synechia) or, less commonly, to the peripheral cornea (anterior synechia). Additionally, granulomatous nodules within the iris stroma may be apparent. Intraocular pressure in the affected eye is initially reduced due to secretory hypotony of the ciliary body. However, as the reaction persists, inflammatory by-products may accumulate in the trabeculum. If this debris builds significantly, and if the ciliary body resumes its normal secretory output, the pressure may rise sharply, resulting in a secondary uveitic glaucoma.

Uveitis is diagnosed by an ophthalmologic examination by a physician or veterinarian.

### Vasculitis

Vasculitis is an inflammation of the blood vessels. Inflammation is a condition in which tissue is damaged by blood cells entering the tissues. These are mostly white blood cells which circulate and serve as our major defense against infection. Ordinarily, white blood cells destroy bacteria and viruses. However, they can also damage normal tissue if they invade it. Vasculitis can affect very small blood vessels (capillaries), medium-size blood vessels (arterioles or venules), or large blood vessels (arteries and veins).

Vasculitis can cause many different symptoms, depending upon what tissues are involved and the severity of the tissue damage. Some patients are not ill and notice occasional spots on their skin. Others are very ill with systemic symptoms and major organ damage. Symptoms include, fever, generally feeling bad ("malaise"), muscle and joint pain, poor appetite, weight loss, fatigue, petechiae, purpura, areas of dead skin can appear as ulcers (especially around the ankles), aching in joints and a frank arthritis with pain, swelling and heat in joints, headaches, behavioral disturbances, confusion, seizures, strokes, numbness and tingling. The diagnosis of vasculitis is based on a person's medical history, current symptoms, a complete physical examination, and the results of specialized laboratory tests. Blood abnormalities which often occur when vasculitis is present include an elevated sedimentation rate, anemia, a high white blood count and a high platelet count. Blood tests can also be used to identify immune complexes or antibodies that cause vasculitis in the circulation and measure whether complement levels are abnormal.

### Atherosclerosis

Atherosclerosis results in the build up of deposits of fatty substances, cholesterol, cellular waste products, and calcium in the inner lining of an artery (*i.e*., plaque) and has a significant inflammatory component. Eventually, this fatty tissue can erode the wall of the artery, diminish its elasticity (stretchiness) and interfere with blood flow. Plaques can also rupture, causing debris to migrate downstream within an artery. This is a common cause of heart attack and stroke. Clots can also form around the plaque deposits, further interfering with blood flow and posing added danger if they break off and travel to the heart, lungs, or brain.

Atherosclerosis often shows no symptoms until flow within a blood vessel has become seriously compromised. Typical symptoms of atherosclerosis include chest pain when a coronary artery is involved, or leg pain when a leg artery is involved. Sometimes symptoms occur only with exertion. In some people, however, they may occur at rest.

Risk factors include smoking, diabetes, obesity, high blood cholesterol, a diet high in fats, and having a personal or family history of heart disease. Cerebrovascular disease, peripheral vascular disease, high blood pressure, and kidney disease involving dialysis are also disorders that may be associated with atherosclerosis.

US 6,025,477 has mentioned antigens indicative of the presence of atherosclerotic plaque, that antibodies can be made thereto and that such antibodies may be used in treating atherosclerosis

### Therapeutic Administration

The invention includes administering to a subject, *e.g*., human or a horse, a composition comprising a CD3 modulator (referred to herein as a "therapeutic compound").

An effective amount of a therapeutic compound is preferably from about 0.1 mg/kg to about 150 mg/kg. Effective doses vary, as recognized by those skilled in the art, depending on route of administration, excipient usage, and coadministration with other therapeutic treatments including use of other anti-inflammatory agents or therapeutic agents for treating, preventing or alleviating a symptom of a particular inflammatory disorder. A therapeutic regimen is carried out by identifying a mammal, *e.g*., a human patient or equine suffering from (or at risk of developing) an inflammatory disorder, or autoimmune disorders using standard methods.

The pharmaceutical compound is administered to such an individual using methods known in the art. Preferably, the compound is administered orally, rectally, nasally, topically or parenterally, *e.g*., subcutaneously, intraperitoneally, intramuscularly, and intravenously. The compound is administered prophylactically, or after the detection of an inflammatory event such as an asthma attack or an allergic reaction. The compound is optionally formulated as a component of a cocktail of therapeutic drugs to treat inflammatory disorders. Examples of formulations suitable for parenteral administration include aqueous solutions of the active agent in an isotonic saline solution, a 5% glucose solution, or another standard pharmaceutically acceptable excipient. Standard solubilizing agents such as PVP or cyclodextrins are also utilized as pharmaceutical excipients for delivery of the therapeutic compounds.

The therapeutic compounds described herein are formulated into compositions for other routes of administration utilizing conventional methods. For example, a therapeutic compound inhibitor is formulated in a capsule or a tablet for oral administration. Capsules may contain any standard pharmaceutically acceptable materials such as gelatin or cellulose. Tablets may be formulated in accordance with conventional procedures by compressing mixtures of a therapeutic compound with a solid carrier and a lubricant. Examples of solid carriers include starch and sugar bentonite. The compound is administered in the form of a hard shell tablet or a capsule containing a binder, *e.g*., lactose or mannitol, a conventional filler, and a tableting agent. Other formulations include an ointment, suppository, paste, spray, patch, cream, gel, resorbable sponge, or foam. Such formulations are produced using methods well known in the art.

Therapeutic compounds are effective upon direct contact of the compound with the affected tissue. Accordingly, the compound is administered topically. For example, to treat contact dermatitis the compound is applied to the area of skin affected. Alternatively, a therapeutic compound is administered systemically. Additionally, compounds are administered by implanting (either directly into an organ such as the intestine, or liver or subcutaneously) a solid or resorbable matrix which slowly releases the compound into adjacent and surrounding tissues of the subject.

For example, for the treatment of gastrointestinal inflammatory disorders, the compound is systemically administered or locally administered directly into gastric tissue. The systemic administration compound is administered intravenously, rectally or orally. For local administration, a compound-impregnated wafer or resorbable sponge is placed in direct contact with gastric tissue. The compound or mixture of compounds is slowly released *in vivo* by diffusion of the drug from the wafer and erosion of the polymer matrix.

Inflammation of the liver (*i.e*., hepatitis) is treated for example by infusing into the liver vasculature a solution containing the compound. Intraperitoneal infusion or lavage is useful to reduce generalized intraperitoneal inflammation of prevent inflammation following a surgical event.

For the treatment of neurological inflammation the compound is administered intravenously or intrathecally (*i.e*., by direct infusion into the cerebrospinal fluid). For local administration, a compound-impregnated wafer or resorbable sponge is placed in direct contact with CNS tissue. The compound or mixture of compounds is slowly released *in vivo* by diffusion of the drug from the wafer and erosion of the polymer matrix. Alternatively, the compound is infused into the brain or cerebrospinal fluid using known methods. For example, a burr hole ring with a catheter for use as an injection port is positioned to engage the skull at a burr hole drilled into the skull. A fluid reservoir connected to the catheter is accessed by a needle or stylet inserted through a septum positioned over the top of the burr hole ring. A catheter assembly (*e.g*., an assembly described in U.S. Patent No. 5,954,687) provides a fluid flow path suitable for the transfer of fluids to or from selected location at, near or within the brain to allow administration of the drug over a period of time.

For treatment of cardiac inflammation, the compound is delivered for example to the cardiac tissue by direct intracoronary injection through the chest wall or using standard percutaneous catheter based methods under fluoroscopic guidance for direct injection into tissue such as the myocardium or infusion of an inhibitor from a stent or catheter which is inserted into a bodily lumen. Any variety of coronary catheter, or a perfusion catheter, is used to administer the compound. Alternatively, the compound is coated or impregnated on a stent that is placed in a coronary vessel.

Pulmonary inflammation is treated for example by administering the compound by inhalation. The compounds are delivered in the form of an aerosol spray from pressured container or dispenser which contains a suitable propellant, *e.g*., a gas such as carbon dioxide, or a nebulizer.

Ocular inflammation is treated, for example, by administering the compound topically to the eye. The compounds are delivered, for example, in a form a gel, liquid, or ointment. Ointments, gels or droppable liquids may be delivered by ocular delivery systems known to the art such as applicators or eyedroppers. Alternatively, the compounds are delivered in an polymer implant that is placed under the under the conjunctiva of the eye. Optionally, the compounds are delivered systemically.

Vasculitis is treated via a route specific for the effected area. In general, vasculitis treated systemically by intravenous administration of the CD3 modulator.

The CD3 modulators are also administered with in conjunction with one or more additional therapeutic compound such as an anti-inflammatory agent or an immunosuppressive agent. For example the CD3 modulator are administered in combination with any of a variety of known therapies for the treatment of autoimmune diseases and/or inflammatory disorders. Suitable known therapies for the treatment of autoimmune diseases and/or inflammatory disorders for use with methods of the invention include, but are not limited to, methotrexate, Cyclosporin A (including, for example, cyclosporin microemulsion and tacrolimus), corticosteroids, statins, interferon beta, nonsteroidal anti-inflammatory drugs (NSAIDs) and the disease-modifying anti-rheumatic drugs (DMARDs). The additional therapeutic is administered prior to, after or concomitantly with administration of a CD3 modulator.

The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected). "Combination therapy" may, but generally is not, intended to encompass the administration of two or more of these therapeutic agents as part of separate monotherapy regimens that incidentally and arbitrarily result in the combinations of the present invention. "Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents.

The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. The sequence in which the therapeutic agents are administered is not narrowly critical. "Combination therapy" also can embrace the administration of the therapeutic agents as described above in further combination with other biologically active ingredients and non-drug therapies (*e.g*., surgery or radiation treatment.) Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

To evaluate whether a patient is benefiting from the administration of an anti-CD3 antibody, alone or in combination, one would examine the patient's symptoms and/or immune response in a quantitative way, and compare the patient's symptoms and/or immune response before and after treatment with the antibody. For example, a patient's symptom can be determined by measuring a particular symptom, or set of symptoms, in a patient before and after treatment with an anti-CD3 antibody. For example, one could measure and monitor symptoms such as fever, joint pain, muscle weakness using any of the standard measurement techniques known in the art. In a successful treatment, the patient status will have improved *(i.e.,* the measurement number will have decreased, or the time to sustained progression will have increased.).

For example, in the treatment of uveitis, anti-CD3 antibodies can be administered in conjunction with, *e.g*., corticosteroids, methotrexate, Cyclosporin A, Cyclophosphamide and/or statins. Likewise, patients afflicted with a disease such as Crohn's Disease or psoriasis can be treated with a combination of an anti-CD3 antibody of the invention and Remicaid (Infliximab), and/or Humira (Adalimumab).

In the treatment of rheumatoid arthritis, the anti-CD3 antibodies are co-administered with corticosteroids, methotrexate, Cyclosporin A, statins, Remicade (Infliximab), Enbrel (Etanercept) and/or Humira (Adalimumab).

Patients with multiple sclerosis can receive a combination of an anti-CD3 with, *e.g*., Glatiramer acetate (Copaxone), Interferon beta-1a (Avonex), Interferon beta-1a (Rebif), Interferon beta-1b (Betaseron or Betaferon), Mitoxantrone (Novantrone), Dexamethasone (Decadron), Methylprednisolone (Depo-Medrol), and/or Prednisone (Deltasone) and/or statins.

Patients with Type I diabetes or Latent Autoimmune Diabetes in the Adult (LADA), are also administered a second agent, such as, for example, GLP-1 or a beta cell resting compound (*i.e.,* a compound that reduces or otherwise inhibits insulin release, such as potassium channel openers).

In addition, CD3 modulators are administered either prophylactically (*i.e*., prior to the onset of an autoimmune disease and/or inflammatory disorder) or therapeutically (*i.e*., during the course of an autoimmune disease and/or inflammatory disorder) to the patient.

In the methods of the invention, the modulators are administered in a therapeutically effective amount. A "therapeutically effective amount" of a CD3 modulator refers to the amount needed to achieve a therapeutic objective, such as the treatment of an autoimmune disease and/or inflammatory disorder, or alleviating a symptom associated with an autoimmune disease and/or inflammatory disorder.

All publications and patent documents cited herein are incorporated herein by reference as if each such publication or document was specifically and individually indicated to be incorporated herein by reference. Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.

### EXAMPLES

### REFERENCE EXAMPLE 1: Anti-CD3 Antibodies in the Treatment of Uveitis

Anti-CD3 antibodies of the disclosure include any of a variety of monoclonal antibodies (mAb) that recognize CD3, such as, for example, OKT3, SP34, UCHTI or 64.1. (*See e.g.,* June, et al., J. Immunol. 136:3945-3952 (1986); Yang, et al., J. Immunol. 137:1097-1100 (1986); and Hayward, et al., Immunol. 64:87-92 (1988)).

A purified mouse anti-rat CD3 monoclonal antibody (BD Pharmingen), referred to as G4.18 antibody, was tested in an inducible rat model of uveitis, as described *e.g*., in Wildner G, Diedrichs-Mohring M, Thurau SR., Eur. J. Immunol. 32(1):299-306 (2002); and in Wildner G, Diedrichs-Mohring M., Int. Immunol. 15(8):927-35 (2003). The G4.18 antibody reacts with T-cell receptor-associated CD3 cell-surface antigen found on thymocytes, peripheral T lymphocytes and dendritic epidermal T cells. (*See e.g.,* Nicolls M.R., et al., Transplantation 55: 459-468 (1993); Nelson, D.J., et al., J. Exp. Med. 179: 203-212 (1994); Morris D.L., and W.J. Komocsar. J. Pharmacol. Toxicol. Methods 37: 37-46 (1997).

To evaluate the effects of anti-CD3 therapy on uveitis, experimental autoimmune uveitis (EAU) was induced in the rats using an intraperitoneal (i.p.) T cell transfer process. In particular, on Day 0, female Lewis rats (weight approximately 140 g each), n = 3 rats/group, received an i.p. injection of 2.4 x. 10⁶ L37 cells transfected with PDSAg-2/3 derived from the retinal autoantigen S-antigen (SAg).

30 minutes before and on days 1, 2, and 3 after transfer of transgenic T cells to induce EAU, rats received i.p. injections of 300 µl of a phosphate buffered saline (PBS) solution (Group 1) or G4.18 antibody in 300 µl of PBS (Group 2). Each group of rats was monitored daily for clinical indicia of uveitis. The clinical grading criteria used herein considered only inflammation in the anterior part of the eye graded using an ophthalmoscope, as described, *e.g*., in de Smet et al., J. Autoimmun., vol. 6: 587 (1993). The clinical grading criteria used in the experiments described herein are shown below in Table A:

**Table A: Clinical Grading Criteria**

| Grade | Description |
|---|---|
| 0.5 | dilated iris vessels, partial inflammatory infiltrates in iris rim and hazy anterior chamber |
| 1.0 | complete circular infiltration of iris rim |
| 2.0 | pupil area completely filled with cells and fibrin |
| 3.0 | formation of hypopyon* |
| 4.0 | anterior chamber completely filled with cells, fibrin and blood |

| | |
|---|---|
| *hypopyon = sedimentation of leukocytes in bottom of anterior chamber; *i.e*., a white lake | |

On day 12, the experiment was terminated, and histological evaluation of retinal destruction was conducted on each rat. The histological criteria described herein considered only the destruction of the retina, as assessed on Day 9 post-treatment cryosections, as described, *e.g*., in de Smet et al., J. Autoimmun., vol. 6:587 (1993). The histological grading criteria used in the experiments described herein are shown below in Table B:

**Table B: Histological Grading Criteria**

| Grade | Description |
|---|---|
| 0.0 | No evidence of inflammatory disease |
| 0.5 | • Trace inflammation, architecture of the retina is grossly intact |
| | • Inflammatory cell infiltration of the retina without evidence of tissue destruction or with outer photoreceptor damage in less than 1/4 of the retina |
| | • Focal non-granulomatous, monocytic infiltration in the choroid, ciliary body and retina |
| 1.0 | • Photoreceptor outer segment damage in ≥ 1/4 of the retina |
| | • Focal areas of destruction with marked dropout of photoreceptors |
| | • Retinal perivascular infiltration and monocytic infiltration in the vitreous |
| 2.0 | • Lesion extending to the outer nuclear layer and in ≥ 1/4 of the retina |
| | • Small exudative retinal detachment |
| | • Mild to moderate number of cells in the vitreous |
| | • Granuloma formation in the uvea and retina |
| | • Occlusive retinal vasculitis, along with serous retinal detachment and loss of photoreceptors |
| 3.0 | • Lesions extending to the inner nuclear layer and in ≥ 1/4 of the retina |
| | • Retinal architecture beginning to be lost, large exudative retinal detachment, moderate to large number of cells in the vitreous |
| | • Formation of Dalen-Fuchs nodules and development of subretinal neovascularization |
| 4.0 • | Full thickness retinal damage in 1/4 of the retina |
| | • Total destruction of retinal architecture |

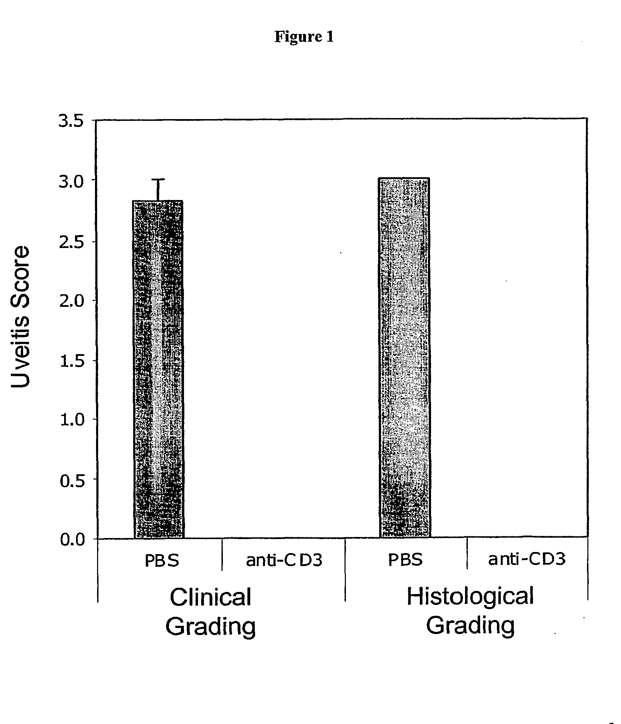

Clinical signs of uveitis began to appear on day 3 of the experiment described herein. Figure 1 is a graph depicting the clinical and histological effects (as determined using the grading criteria described above in Tables A and B) on uveitis progression in rats that received PBS (*i.e*., Group 1) and rats that received the anti-CD3 antibody (*i.e*., Group 2). The results presented herein are reported as the mean of the maximal scores from all eyes of the group from the entire observation time.

### EXAMPLE 2: Anti-CD3 Antibodies in the Treatment of Atherosclerosis

The effects of anti-CD3 antibodies in the treatment of atherosclerosis were evaluated using a hamster anti-mouse CD3 antibody. The experiments described herein were designed to evaluate the effect of anti-CD3 therapy on the development of atherosclerotic plaques, as well as the effect of anti-CD3 therapy on the progression of established atherosclerotic lesions.

A cell line producing F(ab')₂ fragments of the hamster 145 2C11 monoclonal antibody that binds to mouse CD3ε (*i.e.,* to the epsilon chain of the murine CD3 complex) was used to evaluate the effects of anti-CD3 therapy. 10-week old male LDLR-/- C57BL/6 mice were used as a model of *in vivo* atherosclerosis. For histological and atherosclerotic plaque development analysis, as well as proliferation and cytokines analysis, littermate mice were fed with high-cholesterol diet (1.25 % cholesterol, 0 % cholate) for 13 weeks or 24 weeks. Anti-CD3 F(ab')₂ (50 µg/mouse/day) was administered intravenously (i.v.) on 5 consecutive days beginning 1 week before (n = 5) (*i.e.,* to evaluate the effect of the anti-CD3 antibody on the development of atherosclerotic plaques) or 13 weeks (n = 9) after initiating the high cholesterol diet (*i.e.,* to evaluate the effect of the anti-CD3 antibody on the progression of established atherosclerotic lesions). Control mice (*n* = 5 for 13 weeks diet; n = 6 for 24 weeks diet) were injected in parallel with PBS.

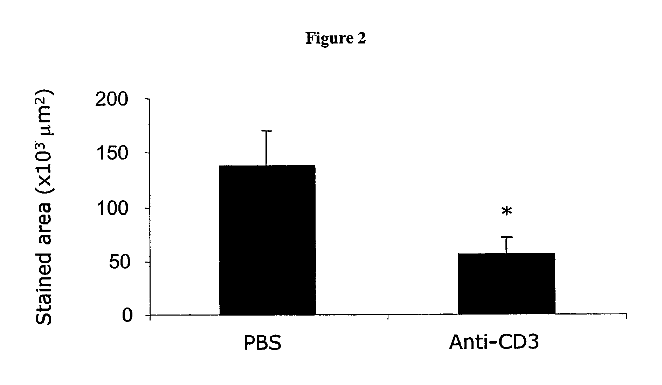

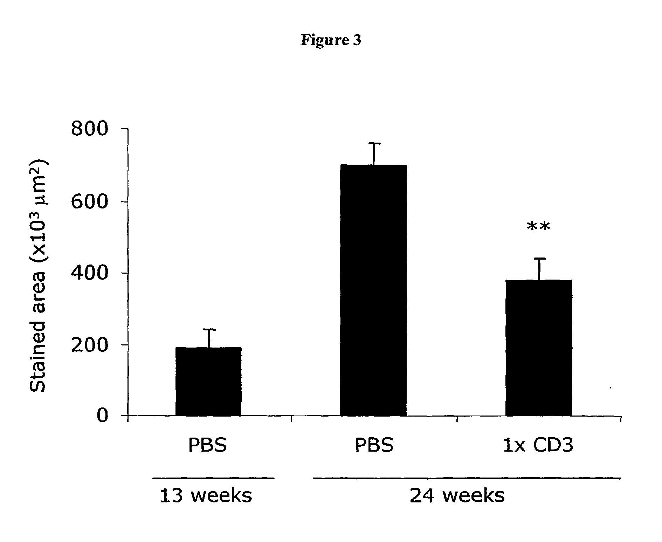

Atherosclerotic lesions within the thoraco-abdominal aorta and aortic sinus were analyzed by Sudan IV staining for lipid deposition (Figures 2 and 3). An average of lipid deposition from 6 sections (5 µm) separated by 50 µm from each other was calculated for each aortic root. Quantification of lipid deposition was performed by computer image analysis using the MetaMorph6 software (Zeiss). The results presented herein are expressed as mean ± s.e.m. Differences between the values were considered significant at P < 0.05 using the two-tailed Student's T-test.

As shown in Figure 2, anti-CD3 therapy inhibited atherosclerotic plaque development in the mice. In addition, Figure 3 demonstrates that anti-CD3 therapy also reduced (*i.e*., inhibited) the progression of atherosclerotic plaque development in established lesions.

## Claims

1. Use of an anti-CD3 antibody for the manufacture of a medicament for treating or alleviating a symptom of an inflammatory disorder in a subject, wherein said inflammatory disorder is atherosclerosis.

2. The use of claim 1, wherein said antibody is a monoclonal antibody.

3. The use of claim 2, wherein said monoclonal antibody is a fully human monoclonal antibody.

4. The use of claim 1, wherein the medicament is for the treatment of a human or an equine.

5. The use of claim 1, wherein said treating or alleviating a symptom further comprises administering to said subject an anti-inflammatory agent or an immunosuppressive agent.

6. The use of claim 5, wherein said anti-inflammatory agent or immunosuppressive agent is a corticosteroid, a statin, interferon beta, a nonsteroidal anti-inflammatory drug (NSAID), methotrexate, Cyclosporin A or a disease-modifying anti-rheumatic drug (DMARD).

7. The use of claim 6, wherein the Cyclosporin A is a cyclosporin microemulsion or tacrolimus.

8. Use of an anti-CD3 antibody for the manufacture of a medicament for reducing the formation of atherosclerotic plaque on an artery.

9. The use of claim 8, wherein said antibody is a monoclonal antibody.

10. The use of claim 9, wherein said monoclonal antibody is a fully human monoclonal antibody.

11. Use of an anti-CD3 antibody in the manufacture of a medicament for inhibiting tissue inflammation, wherein said medicament is to be administered to directly contact an inflamed tissue, and wherein said tissue is vascular tissue.

12. The use of claim 11, wherein said antibody is a monoclonal antibody.

13. The use of claim 12, wherein said monoclonal antibody is a fully human monoclonal antibody.

14. An anti-CD3 antibody for use in treating or alleviating a symptom of an inflammatory disorder, wherein said inflammatory disorder is atherosclerosis.

15. An anti-CD3 antibody for use in reducing the formation of atherosclerotic plaque on an artery.

16. An anti-CD3 antibody for use in inhibiting tissue inflammation, wherein said tissue is vascular tissue, and wherein said tissue is exposed to said anti-CD3 antibody.

17. The anti-CD3 antibody of any of claims 14 to 16, wherein said antibody is a monoclonal antibody.

18. The anti-CD3 antibody of claim 17, wherein said monoclonal antibody is a fully human monoclonal antibody.

19. The anti-CD3 antibody of claim 14, wherein said treating or alleviating a symptom of an inflammatory disorder further comprises administering an anti-inflammatory agent or an immunosuppressive agent to the subject.

## Patentansprüche

1. Verwendung eines anti-CD3-Antikörpers zur Herstellung eines Arzneimittels zur Behandlung oder Linderung eines Symptoms einer entzündlichen Krankheit bei einem Individuum, wobei die entzündliche Krankheit Atherosklerose ist.

2. Verwendung nach Anspruch 1, wobei der Antikörper ein monoklonaler Antikörper ist.

3. Verwendung nach Anspruch 2, wobei der monoklonale Antikörper ein vollständig menschlicher monoklonaler Antikörper ist.

4. Verwendung nach Anspruch 1, wobei das Arzneimittel zur Behandlung eines Menschen oder eines Pferds ist.

5. Verwendung nach Anspruch 1, wobei das Behandeln oder Lindern eines Symptoms außerdem ein Verabreichen eines entzündungshemmenden Mittels oder eines immunsuppressiven Mittels an das Individuum umfasst.

6. Verwendung nach Anspruch 5, wobei das entzündungshemmende Mittel oder das immunsuppressive Mittel ein Corticosteroid, ein Statin, Interferon beta, ein nicht-steroidales entzündungshemmendes Arzneimittel (NSAID), Methotrexat, Cyclosporin A oder ein Krankheits-veränderndes anti-rheumatisches Arzneimittel (DMARD) ist.

7. Verwendung nach Anspruch 6, wobei das Cyclosporin A eine Cyclosporin-Mikroemulsion oder Tacrolimus ist.

8. Verwendung eines anti-CD3-Antikörpers zur Herstellung eines Arzneimittels zur Verringerung der Bildung atherosklerotischer Plaque an einer Arterie.

9. Verwendung nach Anspruch 8, wobei der Antikörper ein monoklonaler Antikörper ist.

10. Verwendung nach Anspruch 9, wobei der monoklonale Antikörper ein vollständig menschlicher monoklonaler Antikörper ist.

11. Verwendung eines anti-CD3-Antikörpers zur Herstellung eines Arzneimittels zur Hemmung von Gewebe-Entzündung, wobei das Arzneimittel so zu verabreichen ist, dass es ein entzündetes Gewebe direkt berührt, und wobei das Gewebe Gefäßgewebe ist.

12. Verwendung nach Anspruch 11, wobei der Antikörper ein monoklonaler Antikörper ist.

13. Verwendung nach Anspruch 12, wobei der monoklonale Antikörper ein vollständig menschlicher monoklonaler Antikörper ist.

14. Anti-CD3-Antikörper zur Verwendung bei der Behandlung oder Linderung eines Symptoms einer entzündlichen Krankheit, wobei die entzündliche Krankheit Atherosklerose ist.

15. Anti-CD3-Antikörper zur Verwendung bei der Verringerung der Bildung von atherosklerotischer Plaque an einer Arterie.

16. Anti-CD3-Antikörper zur Verwendung bei der Hemmung von Gewebe-Entzündung, wobei das Gewebe Gefäßgewebe ist, und wobei das Gewebe dem anti-CD3-Antikörper ausgesetzt wird.

17. Anti-CD3-Antikörper nach einem der Ansprüche 14 bis 16, wobei der Antikörper ein monoklonaler Antikörper ist.

18. Anti-CD3-Antikörper nach Anspruch 17, wobei der monoklonale Antikörper ein vollständig menschlicher monoklonaler Antikörper ist.

19. Anti-CD3-Antikörper nach Anspruch 14, wobei das Behandeln oder Lindern eines Symptoms einer entzündlichen Krankheit außerdem ein Verabreichen eines entzündungshemmenden Mittels oder eines immunsuppressiven Mittels an das Individuum umfasst.

## Revendications

1. Utilisation d'un anticorps anti-CD3 pour la fabrication d'un médicament pour le traitement ou l'atténuation d'un symptôme d'un trouble inflammatoire chez un sujet, dans laquelle ledit trouble inflammatoire est l'athérosclérose.

2. Utilisation selon la revendication 1, dans laquelle ledit anticorps est un anticorps monoclonal.

3. Utilisation selon la revendication 2, dans laquelle ledit anticorps monoclonal est un anticorps monoclonal entièrement humain.

4. Utilisation selon la revendication 1, dans laquelle le médicament est pour le traitement d'un humain ou d'un équidé.

5. Utilisation selon la revendication 1, dans laquelle le traitement ou l'atténuation d'un symptôme comprend en outre l'administration audit sujet d'un agent anti-inflammatoire ou d'un agent immunodépresseur.

6. Utilisation selon la revendication 5, dans laquelle ledit agent anti-inflammatoire ou agent immunodépresseur est un corticostéroïde, une statine, l'interféron bêta, un médicament anti-inflammatoire non stéroïdien (AINS), le méthotrexate, la cyclosporine A ou un agent antirhumatismal modificateur de la maladie (ARMM).

7. Utilisation selon la revendication 6, dans laquelle la cyclosporine A est une microémulsion de cyclosporine ou le tacrolimus.

8. Utilisation d'un anticorps anti-CD3 pour la fabrication d'un médicament pour réduire la formation de plaque athérosclérotique sur une artère.

9. Utilisation selon la revendication 8, dans laquelle ledit anticorps est un anticorps monoclonal.

10. Utilisation selon la revendication 9, dans laquelle ledit anticorps monoclonal est un anticorps monoclonal entièrement humain.

11. Utilisation d'un anticorps anti-CD3 dans la fabrication d'un médicament pour inhiber une inflammation tissulaire, dans laquelle ledit médicament doit être administré de manière à venir en contact direct avec un tissu enflammé, et dans laquelle ledit tissu est un tissu vasculaire.

12. Utilisation selon la revendication 11, dans laquelle ledit anticorps est un anticorps monoclonal.

13. Utilisation selon la revendication 12, dans laquelle ledit anticorps monoclonal est un anticorps monoclonal entièrement humain.

14. Anticorps anti-CD3 pour utilisation dans le traitement ou l'atténuation d'un symptôme d'un trouble inflammatoire, ledit trouble inflammatoire étant l'athérosclérose.

15. Anticorps anti-CD3 pour utilisation dans la réduction de la formation de plaque athérosclérotique sur une artère.

16. Anticorps anti-CD3 pour utilisation dans l'inhibition d'une inflammation tissulaire, dans lequel ledit tissu est un tissu vasculaire, et dans lequel tissu est exposé audit anticorps anti-CD3.

17. Anticorps anti-CD3 selon l'une quelconque des revendications 14 à 16, lequel anticorps est un anticorps monoclonal.

18. Anticorps anti-CD3 selon la revendication 17, lequel anticorps est un anticorps monoclonal entièrement humain.

19. Anticorps anti-CD3 selon la revendication 14, ledit traitement ou ladite atténuation d'un symptôme d'un trouble inflammatoire comprenant en outre l'administration au sujet d'un agent anti-inflammatoire ou d'un agent immunodépresseur.
